# EUROPÄISCHE PATENTSCHRIFT

(11) **EP 1 226 782 B1**
(45) Veröffentlichungstag und Bekanntmachung des Hinweises auf die Patenterteilung: **08.11.2006**
(21) Anmeldenummer: 02000804.1
(22) Anmeldetag: 14.01.2002
(51) Int. Cl.: A61B 5/00

(54) **Verfahren und Vorrichtung zur poststationären Überwachung eines Patienten**
Method and device for post-operative monitoring of a patient
Méthode et dispositif de surveillance post-opératoire d'un patient

(30) Priorität: 25.01.2001 DE 10103325
(43) Veröffentlichungstag der Anmeldung: 31.07.2002
(73) Patentinhaber: SIEMENS AKTIENGESELLSCHAFT, 80333 München (DE)
(72) Erfinder: Christ, Tilo, 91058 Erlangen (DE); Schmidt, Volker, Dr., 91054 Erlangen (DE); Schüll, Hans, 91085 Weisendorf (DE); Striebel, Werner, 90592 Schwarzenbruck (DE)

(56) Entgegenhaltungen:
- EP-A- 1 034 734
- EP-A- 1 101 437
- WO-A-98/24212
- FR-A- 2 717 332
- FR-A- 2 750 236
- US-A- 5 339 821
- US-A- 5 416 695
- US-A- 5 544 661
- US-A- 5 553 609
- US-A- 5 967 975

## Beschreibung

Die Erfindung betrifft ein Verfahren und eine Vorrichtung zur poststationären Überwachung eines Patienten.

Nach einer Operation eines Patienten können typische postoperative Komplikationen auftreten, die u.a. Pneumonien, Lungenentzündungen und Bronchitis, Harnwegsinfekte, Nachblutungen, Bein- und Beckenvenenthrombosen, Blutzuckerentgleisungen und Wundheilstörungen umfassen. Bei einer derzeitigen Tendenz, einen Krankenhausaufenthalt eines Patienten zu verkürzen, werden auch Patienten nach einer Operation früher aus dem Krankenhaus entlassen. Dadurch besteht das Risiko, eventuell eintretende postoperative Komplikationen zu spät zu erkennen.

Mittels eines in der US 5,967,975 beschriebenen Systems kann überwacht werden, ob ein Patient daheim und zu vorbestimmten Zeiten seinen Gesundheitszustand wie verordnet selber überwacht, indem er Gesundheitsparameter wie seinen Blutdruck, Blutzucker oder Blutgerinnungsfaktor mit einem geeigneten medizintechnischen Gerät selber bestimmt. Das System umfasst ein Endgerät, an das das medizintechnische Gerät angeschlossen werden kann, um mit dem medizintechnischen Gerät ermittelte Messwerte aufzunehmen. Das Endgerät überprüft daraufhin, ob die Messwerte bedenklich oder unbedenklich sind, indem es die Messwerte mit vorgegeben Referenzwerten vergleicht und übermittelt vorgefertigte, den Messwerten entsprechenden Nachrichten über eine Kommunikationsverbindung an eine zentrale Überwachungsstation. Die zentrale Überwachungsstation empfängt diese Nachrichten, überprüft, ob von dem Endgerät innerhalb vorbestimmter Zeitfenster eine Nachricht empfangen wurde, und speichert die empfangenen Nachrichten. Das System umfasst ebenfalls eine Alarmeinrichtung.

Aus der WO 93/01574 ist ein Alarmsystem zur Benachrichtigung von Klinikpersonal, dass Geräte zur Lebenserhaltung eines Patienten sofort überprüft werden müssen, bekannt. Das System umfasst einen Alarmgeber, der dem Patienten und den Geräten zugeordnete Signale generiert. Die Signale werden einer Zentralstation zugeführt. Die Zentralstation leitet die empfangenen Signale gleichzeitig an Pager, die daraufhin vibrieren und eine Information über den Patienten und den Geräten anzeigen, weiter.

Aus der EP 1 101 437 ist eine Vorrichtung zur poststationären Überwachung eines Patienten bekannt, die während der Überwachung am Patienten erfasste Daten an zentralem Ort speichert und auswertet. Wird ein kritisches Auswertungsergebnis erhalten, erzeugt die Vorrichtung ein Alarmsignal für eine Überwachungsperson. Die Übermittlung der Patientendaten zur zentralen Überwachungsvorrichtung kann beispielsweise über Internet, Fax oder Telefonnetz erfolgen. Dazu sind Übertragungsvorrichtungen wie beispielsweise ein Computer, ein Faxgerät oder ein Telefonmodulator erforderlich.

Eine solche Vorrichtung wird auch in US-A-5,553,609 beschrieben

Der Erfindung liegt daher die Aufgabe zugrunde, ein Verfahren anzugeben sowie eine Vorrichtung derart auszubilden, mit deren bzw. dessen Hilfe Voraussetzungen geschaffen werden, dass insbesondere postoperative Komplikationen zuverlässig und frühzeitig erkannt werden, obwohl der Patient frühzeitig aus dem Krankenhaus entlassen wurde, und bei dem der Patient die für die Überwachung relevanten Daten ohne eine eigens dafür erforderliche Übertragungsvorrichtung übermitteln kann.

Nach der Erfindung wird diese Aufgabe gelöst durch ein Verfahren zur poststationären Überwachung eines Patienten zum Erkennen einer Pneumonie, einer Nachblutung, einer Wundheilungsstörung, einer Lungenkomplikation, einer Harnwegsinfektionen oder einer Thrombose des Patienten, für dessen Ausführung eine Datenbank vorgesehen ist, welche an einem Ort angeordnet ist, der von dem Ort, an dem sich der Patient während der Überwachung aufhält, verschieden ist, aufweisend folgende Verfahrensschritte:
- Erfassen relevanter Daten des Patienten für dessen poststationäre Überwachung und
- interaktives Übermitteln der relevanten Daten an die Datenbank mittels Spracheingabe durch den Patienten.
   Erfindungsgemäß wird der Patient also poststationär überwacht, um eine Pneumonie, eine Nachblutung, eine Wundheilungsstörung, eine Lungenkomplikation, eine Harnwegsinfektion oder eine Thrombose, also typische postoperative Komplikationen, frühzeitig zu erkennen. Durch die poststationäre, also außerklinische Überwachung des Patienten werden insbesondere Pflegekosten gespart. Der Patient versorgt sich z.B. in seiner Wohnung selbst oder wird beispielsweise von einem Angehörigen oder einem ambulanten Pflegedienst versorgt. Um eine ordnungsgemäße poststationäre Überwachung des Patienten sicher zu stellen, erfasst der Patient oder eine weitere Person zum Erkennen der typischen Komplikationen relevante Daten des Patienten. Diese Daten umfassen u.a. zum Erkennen einer Nachblutung den Zeitpunkt des Entdeckens der Nachblutung, Zentralisationszeichen, Blutdruck, Herzfrequenz oder sichtbare Blutungen. Für eine Wundheilungsstörung umfassen die relevanten Daten u.a. Schmerzen, Überwärmung, Rötung, Einschränkung der Beweglichkeit und eine Blutzuckerentgleisung des Patienten. Für eine Lungenkomplikation relevante Daten umfassen Auswurf, Husten oder Fieber. Für eine Harnwegsinfektion umfassen die relevanten Daten Beschwerden beim Wasserlassen, Nykturie oder Pollakisurie und für eine Thrombose umfassen die relevante Daten Schmerzen in Wade oder Bein, Operationstyp, Beinumfänge im Seitenvergleich und im Verlauf und Beintemperaturverlauf. Die Beinumfänge können z.B. mit einem Maßband, die Überwärmung mit einer Infrarotkamera oder einem Hautthermometer und das Fieber mit einem Fieberthermometer gemessen werden. Außerdem können dem Patienten während der Überwachung ein Blutzuckermessgerät, Urinsticks, ein Blutdruckmessgerät oder eine zum Überwachen einer Wunde vorgesehene Digitalkamera zur Verfügung gestellt werden. Nach dem Erfassen der relevanten Daten werden diese an die zentrale Datenbank übermittelt, die z.B. in dem Krankenhaus angeordnet ist, in dem der Patient behandelt wurde. Folglich hat ein den Patienten behandelnder Arzt leicht Zugang zu den relevanten Daten. Somit sind Voraussetzungen gegeben, Komplikationen frühzeitig und zuverlässig zu erkennen, obwohl der Patient nicht stationär behandelt wird.
   Nach einer bevorzugten Variante der Erfindung wird die Datenbank von einem Service-Anbieter betrieben. Ein solcher Service-Anbieter kann in besonders günstiger Weise die Datenbank pflegen oder weitere Dienste anbieten. Insbesondere wenn dem Service-Anbieter gemäß einer Variante der Erfindung ein Call-Center zugeordnet ist, können Fragen des Patienten oder des Arztes an das Call-Center gerichtet werden. Folglich kann eine Betreuung des Patienten effektiver gestaltet und bei Fragen schnell, freundlich und zuverlässig weiter geholfen werden.
   Bei einer weiteren vorteilhaften Ausführungsform der Erfindung werden die relevanten Daten mittels eines Fragebogens erfasst. Dieser Fragebogen kann beispielsweise in Papierform dem Patienten bei der Entlassung aus dem Krankenhaus mitgegeben werden oder ist auf einer dem Patienten zugeordneten und beispielsweise von dem Service-Anbieter gepflegten WWW-Homepage abrufbar. Der Fragebogen kann auch als Check-Liste dienen, damit der Patient oder die weitere Person alle relevanten Daten ermittelt.
   Gemäß einer Ausführungsform der Erfindung umfassen die relevanten Daten auch eine Beschreibung des Allgemeinzustandes des Patienten. Der Allgemeinzustand des Patienten lässt sich beispielsweise durch Ermitteln der körperlichen Leistungsfähigkeit, beispielsweise beim Treppensteigen, überprüfen. Der Appetit, die Temperatur, der Stuhlgang, der Puls, der Blutdruck oder der Blutzucker des Patienten lassen ebenfalls Rückschlüsse auf den Allgemeinzustand des Patienten zu. Somit können die relevanten Daten insbesondere eine Beschreibung des Allgemeinzustandes in Kombination mit Daten zum Erkennen einer typischen Komplikation umfassen. Eine typische Komplikation nach einer Allgemeinnarkose mit Intubation ist beispielsweise eine Lungenentzündung. Folglich umfassen die relevanten Daten nach einer Allgemeinnarkose mit Intubation insbesondere Daten zum Erkennen einer Lungenkomplikation und Informationen über den Allgemeinzustand des Patienten.
   Gemäß einer weiteren Ausführungsform der Erfindung werden die relevanten Daten mit einem Telefon, einem an das Internet anschließbaren Rechner oder einem Faxgerät zumindest indirekt an die Datenbank übermittelt. Insbesondere bei einer Übermittlung per Telefon muss dem Patienten während der poststationären Überwachung kein kompliziertes und eventuell kostspieligen Übertragungsgerät zur Verfügung gestellt werden, da in der Regel jeder Haushalt mit einem Telefon ausgestattet ist. Ein geeignet ausgeführtes Messgerät kann auch an den Rechner angeschlossen werden. Folglich können die mit dem Messgerät ermittelten Werte mit dem Rechner an die Datenbank übermittelt und Ablesefehler vermieden werden.
   Wenn nach einer bevorzugten Ausführungsform der Erfindung die Datenbank über das Internet abgefragt werden kann, hat beispielsweise der den Patienten behandelnde Arzt leichten Zugang auf die relevanten Daten und kann somit frühzeitig und zuverlässig eventuell anfallende Komplikationen des Patienten entdecken und nötigenfalls Hilfsmaßnahmen einleiten.
   Bei einer weiteren Variante der Erfindung ist vorgesehen, dass die Datenbank bei einem Ausbleiben der relevanten Daten den Patienten und/oder wenigstens eine weitere Person automatisch benachrichtigt. Die weitere Person ist beispielsweise der den Patienten behandelnde Arzt. Somit ist eine Voraussetzung geschaffen, dass der Patient an die Wichtigkeit der Überwachung erinnert werden kann, insbesondere wenn er die Ermittlung oder die Übertragung der relevanten Daten versehentlich vergaß, oder dass der Arzt bei einer Unterbrechung der Überwachung schnell und zuverlässig informiert wird.
   Nach einer weiteren vorteilhaften Ausführungsform der Erfindung werden die relevanten Daten insbesondere automatisch mit einer der Datenbank zugeordneten Auswerteeinrichtung ausgewertet. Die Auswerteeinrichtung kann insbesondere ein Expertensystem aufweisen, welches die relevanten Daten kontextabhängig interpretiert und beispielsweise bei unklaren Situationen beim Patienten nachfragt. Unter kontextabhängiger Interpretation wird u.a. verstanden, dass die relevanten Daten mit entsprechenden und einem Patienten, der keine postoperativen Komplikationen hatte, zugeordneten Daten verglichen werden. Ein Vorteil einer automatisierten Auswertung ist das Einsparen von Personal. Außerdem ist somit sicher gestellt, dass ein Auswerten der relevanten Daten nicht versehentlich übersehen wird.
   Bei einer besonders vorteilhaften Ausführung der Erfindung ist vorgesehen, dass die Datenbank automatisch den Patienten und/oder wenigstens eine weitere Person alarmiert, wenn die ausgewerteten relevanten Daten kritisch sind. Beispielsweise könnte der durchschnittliche Verlauf eines Schmerzmittelverbrauchs nach einer bestimmten Operation mehrerer Patienten, die keine Komplikationen hatten, ermittelt und in der Datenbank gespeichert werden. Der durchschnittliche Verlauf des Schmerzmittelverbrauchs kann dann als Alarmgrenze dienen. Die weitere Person kann z.B. der den Patienten behandelnde Arzt sein, der beispielsweise aufgrund der Alarmierung die Daten überprüft und wenn nötig, schnell und zuverlässig Notmaßnahmen für den Patienten einleiten kann.
   Die Aufgabe wird auch gelöst durch eine Vorrichtung zur poststationären Überwachung eines Patienten zum Erkennen einer Pneumonie, einer Nachblutung, einer Wundheilungsstörung, einer Lungenkomplikation, einer Harnwegsinfektionen oder einer Thrombose des Patienten, aufweisend
- eine Datenbank, welche an einem Ort angeordnet ist, der von dem Ort, an dem sich der Patient während der Überwachung aufhält, verschieden ist und während der poststationären Überwachung erfasste relevante Daten für die poststationäre Überwachung des Patienten speichert,
- eine der Datenbank zugeordnete Auswerteeinrichtung zur Auswertung der relevanten Daten,
- Mittel zur Spracheingabe, die dazu ausgebildet sind, der Datenbank relevante Daten interaktiv sprachlich zu übermitteln, und
- eine Alarmeinrichtung zur Erzeugung eines Alarmsignal, wenn die ausgewerteten relevanten Daten kritisch sind.

Die Vorrichtung ist also dadurch gekennzeichnet, dass insbesondere typische postoperative Komplikationen eines Patienten frühzeitig erkannt werden können, obwohl der Patient insbesondere frühzeitig aus dem Krankenhaus entlassen wurde. Während der poststationären Überwachung erfasst der Patient oder eine weitere Person, wie beispielsweise ein Angehöriger oder eine Pflegeperson, für das Erkennen der Komplikationen relevante Daten und übermittelt diese an eine zentrale Datenbank. Die Datenbank kann beispielsweise in dem Krankenhaus angeordnet sein, in dem der Patient behandelt wurde. Somit braucht einerseits der Patient nicht stationär behandelt zu werden, wodurch Kosten gespart werden, und andererseits hat der den Patienten behandelnde Arzt jederzeit die Möglichkeit, die relevanten Daten einzusehen und dementsprechend Komplikationen frühzeitig zu erkennen und wenn nötig, Hilfsmaßnahmen für den Patienten einzuleiten.

Bei einer vorteilhaften Ausgestaltung der Erfindung ist die Datenbank derart ausgeführt ist, dass sie bei einem Ausbleiben der relevanten Daten automatisch den Patienten und/oder wenigstens eine weitere Person benachrichtigt.

Ein Ausführungsbeispiel der Erfindung ist in den beigelegten schematischen Zeichnungen dargestellt. Es zeigen:
- Fig. 1: ein Schaubild eines erfindungsgemäßen medizinischen Systems und
- Fig. 2: einen Fragebogen.

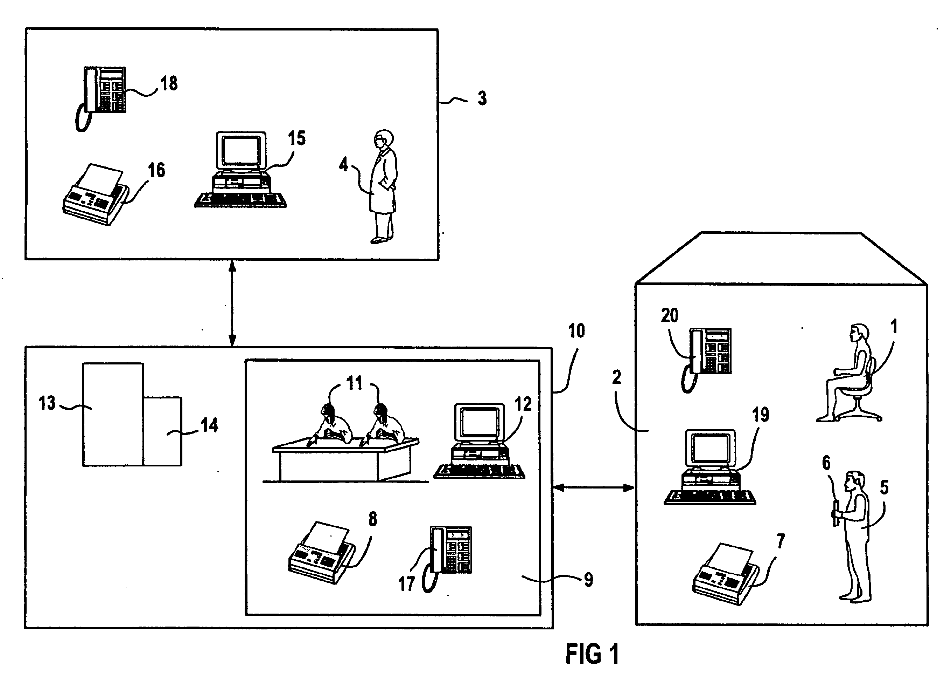

In der Fig. 1 ist schematisch ein Patient 1 in seinem Haus 2 gezeigt, der in einem Krankenhaus 3 operiert und von seinem Arzt 4 nach der Operation nach Hause entlassen wurde. Während der Operation wurde im Falle des vorliegenden Ausführungsbeispieles beim Patienten 1 eine Allgemeinnarkose mit Intubation durchgeführt, wodurch der Patient 1 ein erhöhtes Risiko aufweist, eine Lungenentzündung als typische postoperative Komplikation zu entwickeln. Deshalb wird im Falle des vorliegenden Ausführungsbeispieles der Patent 1 täglich einmal von einer Pflegeperson 5 aufgesucht, die zum Erkennen von Lungenkomplikationen, wie z.B. einer Lungenentzündung als typische postoperative Komplikation, relevante Daten des Patienten 1 ermittelt.

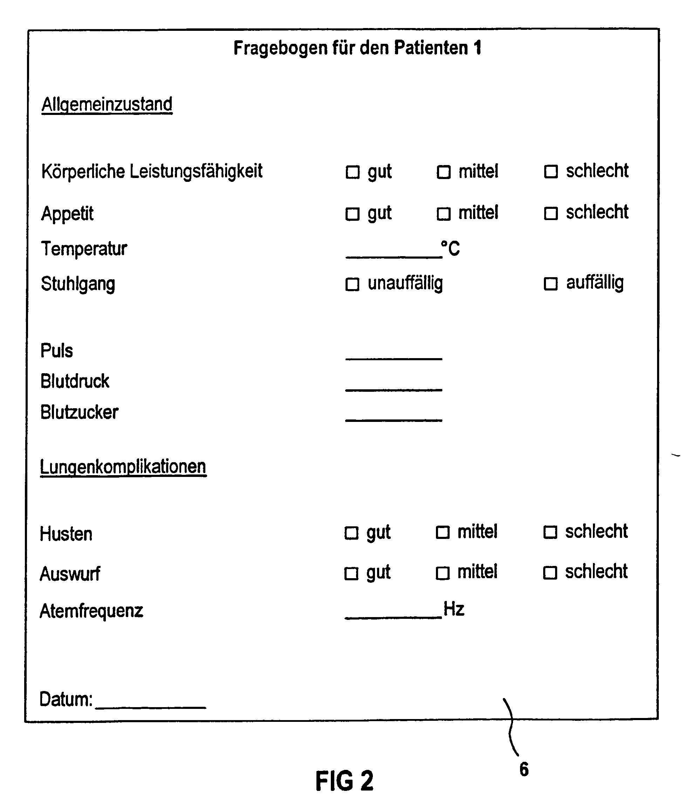

Damit bei der Ermittlung der relevanten Daten nicht versehentlich ein Teil der relevanten Daten vergessen wird, verfügt die Pflegeperson 5 über einen dem Patienten 1 zugeordneten und in der Fig. 2 exemplarisch gezeigten Fragebogen 6, der gleichzeitig als Check-Liste zum Ermitteln der relevanten Daten dient.

Wie in der Fig. 2 gezeigt, umfassen die relevanten Daten im Falle des vorliegenden Ausführungsbeispieles Angaben zum Allgemeinzustand des Patienten 1 und Angaben, die speziell zum Erkennen einer Lungenkomplikation geeignet sind. Um die relevanten Daten zu erhalten, befragt die Pflegeperson 5 den Patienten 1 nach dessen körperlicher Leistungsfähigkeit, dessen Stuhlgang, dessen Appetit oder ermittelt das Auftreten von Husten oder Auswurf und notiert die entsprechenden Antworten auf dem Fragebogen 6. Anschließend ermittelt die Pflegeperson 5 die Temperatur, den Puls, den Blutdruck, den Blutzuckerspiegel und die Atemfrequenz des Patienten 1 mit im Allgemein bekannten und in der Fig. 1 nicht gezeigten Messinstrumenten, wie beispielsweise einem Fieberthermometer, einem Blutdruckmessgerät, Urinsticks, etc. und notiert die entsprechenden Werte auf dem Fragebogen 6.

Anschließend faxt die Pflegeperson 5 den Fragebogen 6 mit einem in dem Haus 2 des Patienten 1 angeordneten Faxgerät 7 an ein Faxgerät 8, welches in einem Call-Center 9 eines Service-Anbieters 10 angeordnet ist. Eine der in dem Call-Center 9 arbeitenden Personen 11 gibt die mittels des gefaxten Fragebogens übermittelten relevanten Daten in einem in dem Call-Center 9 angeordneten Rechner 12 ein, welcher mit einer Datenbank 13 in der Fig. 1 in nicht dargestellten Weise verbunden ist. Die relevanten Daten werden mit der Identität des Patienten 1 und dem Datum, an dem die relevanten Daten ermittelt wurden, in der Datenbank 13 gespeichert.

Im Falle des vorliegenden Ausführungsbeispieles ist der Datenbank 13 eine Auswerteeinrichtung 14 zugeordnet, welche die relevanten Daten insbesondere auf Vollständigkeit und Plausibilität überprüft. Ferner vergleicht die Auswerteeinrichtung 14 die relevanten Daten mit Vergleichswerten, die im Falle des vorliegenden Ausführungsbeispiels einer Person zugeordnet sind, welche keine postoperative Komplikation und insbesondere keine Lungenkomplikation aufweist. Eine Person ohne postoperative Komplikationen weist beispielsweise eine gute bis mittlere körperliche Leistungsfähigkeit und einen guten bis mittleren Appetit auf. Sie hat des weiteren eine normale Temperatur, also kein Fieber, und einen unauffälligen Stuhlgang, normalen Blutdruck usw.. Der Arzt 4 kann, wenn er es für nötig hält, die Vergleichswerte auch selber ändern. Weicht im Falle des vorliegenden Ausführungsbeispiels einer der Werte der relevanten Daten von dem entsprechenden Vergleichswert ab, stuft die Auswerteeinrichtung 14 die relevanten Daten als kritisch ein und die Datenbank 13 schickt automatisch eine e-mail an einen in dem Krankenhaus 3 angeordneten Rechner 15, um den Arzt 4 über die kritischen relevanten Daten zu informieren. Insbesondere bei einer gestörten Internetverbindung kann diese Nachricht dem Arzt 4 mittels des Faxgerätes 8 und eines in dem Krankenhaus 3 angeordneten Faxgerätes 16 oder mittels eines im Call-Center 9 angeordneten Telefons 17 und eines in dem Krankenhaus 3 angeordneten Telefons 18 von einer der Personen 11 mitgeteilt werden.

Im Falle des vorliegenden Ausführungsbeispiels ist es ferner vorgesehen, dass die Datenbank 13 über das Internet kontaktierbar ist. So kann z.B. der Arzt 4 bei einer Benachrichtigung durch die Datenbank 13 bzw. durch einer der Personen 11 die Datenbank 13 mit dem Rechner 15 kontaktieren und die relevanten Daten des Patienten 1 abfragen. Aufgrund dieser Abfrage kann der Arzt 4 eventuell auf eine Lungenkomplikation des Patienten 1 schließen und nötigenfalls Hilfsmaßnahmen für den Patienten 1 einleiten.

Bei Unvollständigkeit oder unplausiblen relevanten Daten benachrichtigt die Datenbank 13 im Falle des vorliegenden Ausführungsbeispiels automatisch den Patienten 1 mit einer e-mail, welche an einen in dem Haus 2 angeordneten und an das Internet angeschlossenen Rechner 19 geschickt wird. Alternativ kann auch eine der Personen 11 den Patienten 1 mittels eines in dem Haus 2 angeordneten Telefons 20 verständigen.

Sollten an einem Tag die relevanten Daten beispielsweise versehentlich nicht an die Datenbank 13 übermittelt werden, informiert die Datenbank 13 automatisch den Arzt 4 und den Patienten 1. Somit ist eine kontinuierliche poststationäre Überwachung des Patienten 1 sichergestellt.

Eine andere Möglichkeit die relevanten Daten des Patienten 1 der Datenbank 13 zu übermitteln, besteht in der Verwendung des an das Internet angeschlossenen Rechners 19. Im Falle des vorliegenden Ausführungsbeispiels kontaktiert der Patient 1 oder die Pflegeperson 5 mit dem Rechner 19 eine dem Patienten 1 zugeordnete und vom Service-Anbieter 10 gepflegte WWW-Homepage. Die WWW-Homepage umfasst den in der Fig. 2 gezeigten Fragebogen 6. Anschließend ermittelt der Patient 1 oder die Pflegeperson 5 die auf dem Fragebogen 6 angegebenen und bereits obenstehend beschriebenen relevanten Daten und trägt sie in den Fragebogen 6 der WWW-Homepage ein. Nachdem die relevanten Daten in dem Fragebogen 6 eingetragen sind, werden sie der Datenbank 13 übermittelt und von der Auswerteeinrichtung 14 ausgewertet.

Eine weitere Möglichkeit der Übermittlung der relevanten Daten besteht darin, dass der Patient 1 oder die Pflegeperson 5 die relevanten Daten telefonisch einer der Personen 11 des Call-Centers 9 übermittelt. Die relevanten Daten des vorliegenden Ausführungsbeispiels sind nur exemplarisch zu verstehen. Die relevanten Daten müssen nicht notwendigerweise eine Beschreibung des Allgemeinzustandes des Patienten 1 umfassen. Die relevanten Daten können auch andere oder weitere Informationen insbesondere über weitere typische postoperative Komplikationen, wie Harnwegsinfekte, Nachblutungen, Bein- und Beckenvenenthrombosen, Blutzuckerentgleisungen und Wundheilstörungen umfassen. Auch das Notieren auf einem Fragebogen 6 ist optional.

Der Patient 1 muss nicht notwendigerweise von einer Pflegeperson 5 überwacht werden. Er kann auch von einem Angehörigen überwacht werden oder kann die relevanten Daten selber ermitteln und zumindest indirekt der Datenbank 13 übermitteln.

Die Auswerteeinrichtung 14 kann auch ein Expertensystem aufweisen. Das Expertensystem kann z.B. die relativen Daten anhand von Regelsystemen oder basierend auf Wahrscheinlichkeiten interpretieren. Dabei ist eine Individualisierung des Expertensystems auf den Patienten 1 vorsehbar, d.h. das Expertensystem lernt den Patienten 1, den es überwacht, im Überwachungsprozess immer besser kennen, es trifft dazu als lernendes System ständig Prognosen über zu erwartende zukünftige relevante Daten, die es mit den wahren relevanten Daten vergleicht. Damit wird eine individualisierte Überwachung erreicht.

Die Datenbank 13 kann auch mit zur Spracheingabe und Sprachwiedergabe geeigneten Mitteln versehen sein. Dann kann der Patient 1 oder die Pflegeperson 5 die Datenbank 13 direkt telefonisch kontaktieren und aufgrund einer interaktiven auf der Datenbank 13 gespeicherten Programmprozedur die relevanten Daten telefonisch der Datenbank 13 übermitteln.

Es können auch Messgeräte zum Ermitteln der relevanten Daten verwendet werden, die an dem Rechner 19 anschließbar sind. Dann ist es möglich, die Messergebnisse, also relevante Daten, direkt an die Datenbank 13 zu übermitteln, wodurch ein eventuelles falsches Ablesen eines Messergebnisses vermieden werden kann.

Der Service-Anbieter 10 muss nicht notwendigerweise über ein Call-Center 9 verfügen. Die Datenbank 13 braucht auch nicht von einem Service-Anbieter 10 betrieben werden. Sie kann beispielsweise auch in dem Krankenhaus 3 angeordnet sein.

Eine Kontaktierung der Datenbank 13 über das Internet ist ebenfalls optional. Obwohl eine automatische Alarmierung des Arztes 4 bei kritischen relevanten Daten wünschenswert ist, ist dieses Merkmal auch optional. Es kann auch eine andere Person oder es können auch weitere Personen alarmiert werden. Die Vergleichswerte können auch anderes als obenstehend beschrieben eingestellt werden. Insbesondere ist es auch denkbar, die Vergleichswerte über die Zeit aus einer Normierung bereits erfasster relevanter Daten zu gewinnen. Beispielsweise könnte der durchschnittliche Verlauf eines Schmerzmittelverbrauchs nach einer bestimmten Operation mehrerer Patienten, die keine Komplikationen hatten, ermittelt und in der Datenbank 13 gespeichert werden. Der durchschnittliche Verlauf des Schmerzmittelverbrauchs kann dann als Vergleichswert dienen.

Die Alarmierung des Patienten 1 oder einer weiteren Person bei einem Ausbleiben der Übermittlung der relevanten Daten an die Datenbank 13 ist ebenfalls optional.

Der Patient 1 muss sich auch nicht notwendigerweise in seinem Haus 2 aufhalten, solange er poststationär überwacht wird.

Die relevanten Daten müssen auch nicht täglich oder generell periodisch ermittelt und/oder übermittelt werden.

Die Überwachung bezieht sich nicht nur ausschließlich auf postoperative Komplikationen sondern generell auch Komplikationen, die ein Patient nach einem Krankenhausaufenthalt haben kann.

## Patentansprüche

1. Verfahren zur poststationären Überwachung eines Patienten zum Erkennen einer Pneumonie, einer Nachblutung, einer Wundheilungsstörung, einer Lungenkomplikation, einer Harnwegsinfektionen oder einer Thrombose des Patienten (1), für dessen Ausführung eine Datenbank (13) vorgesehen ist, welche an einem Ort angeordnet ist, der von dem Ort (2), an dem sich der Patient (1) während der Überwachung aufhält, verschieden ist, aufweisend folgende Verfahrensschritte:
- Erfassen relevanter Daten des Patienten (1) für dessen poststationäre Überwachung und
- interaktives Übermitteln der relevanten Daten an die Datenbank (13) mittels Spracheingabe durch den Patienten.

2. Verfahren zur poststationären Überwachung eines Patienten nach Anspruch 1, bei welchem die Datenbank (13) von einem Service-Anbieter (10) betrieben wird.

3. Verfahren zur poststationären Überwachung eines Patienten nach Anspruch 2, bei welchem dem Service-Anbieter (10) ein Call-Center (9) zugeordnet ist.

4. Verfahren zur poststationären Überwachung eines Patienten nach einem der Ansprüche 1 bis 3, bei welchem die relevanten Daten eine Beschreibung des Allgemeinzustandes des Patienten (1) umfassen.

5. Verfahren zur poststationären Überwachung eines Patienten nach einem der Ansprüche 1 bis 4, bei welchem die Datenbank (13) über das Internet abgefragt werden kann.

6. Verfahren zur poststationären Überwachung eines Patienten nach einem der Ansprüche 1 bis 5, bei welchem die Datenbank (13) bei einem Ausbleiben der relevanten Daten den Patienten (1) und/oder wenigstens eine weitere Person (4) automatisch benachrichtigt.

7. Verfahren zur poststationären Überwachung eines Patienten nach einem der Ansprüche 1 bis 6, bei welchem die relevanten Daten insbesondere automatisch mit einer der Datenbank (13) zugeordneten Auswerteeinrichtung (14) ausgewertet werden.

8. Verfahren zur poststationären Überwachung eines Patienten nach Anspruch 7, bei welchem die Datenbank (13) automatisch den Patienten (1) und/oder wenigstens eine weitere Person (4) alarmiert, wenn die ausgewerteten relevanten Daten kritisch sind.

9. Vorrichtung zur poststationären Überwachung eines Patienten zum Erkennen einer Pneumonie, einer Nachblutung, einer Wundheilungsstörung, einer Lungenkomplikation, einer Harnwegsinfektionen oder einer Thrombose des Patienten (1), aufweisend
- eine Datenbank (13), welche an einem Ort angeordnet ist, der von dem Ort (2), an dem sich der Patient (1) während der Überwachung aufhält, verschieden ist und während der poststationären Überwachung erfasste relevante Daten für die poststationäre Überwachung des Patienten (1) speichert,
- Mittel zur Spracheingabe, die dazu ausgebildet sind, der Datenbank (13) relevante Daten sprachlich interaktiv zu übermitteln,
- eine der Datenbank (13) zugeordnete Auswerteeinrichtung (14) zur Auswertung der relevanten Daten und
- eine Alarmeinrichtung zur Erzeugung eines Alarmsignal, wenn die ausgewerteten relevanten Daten kritisch sind.

10. Vorrichtung zur poststationären Überwachung eines Patienten nach Anspruch 9, bei welcher die Datenbank (13) derart ausgeführt ist, dass sie bei einem Ausbleiben der relevanten Daten automatisch den Patienten (1) und/oder wenigstens eine weitere Person (4) benachrichtigt.

11. Verfahren zur poststationären Überwachung eines Patienten (1), aufweisend folgende Verfahrensschritte:
- Erfassen, am Ort (2) der poststationären Überwachung, relevante Daten, die zum Erkennen einer Pneumonie, einer Nachblutung, einer Wundheilungsstörung, einer Lungenkomplikation, einer Harnwegsinfektionen oder einer Thrombose des Patienten (1) während dessen poststationärer Überwachung geeignet sind,
- interaktives Übermitteln der relevanten Daten über ein Informationsübertragungsnetz an eine zentrale Datenbank (13) mittels Spracheingabe durch den Patienten,
- Auswerten der relevanten Daten mit einer der Datenbank (13) zugeordneten Auswerteeinrichtung (14).

12. Verfahren nach Anspruch 11, bei welchem automatisch der Patient (1) und/oder wenigstens eine weitere Person (4) alarmiert wird, wenn die ausgewerteten relevanten Daten kritisch sind.

## Claims

1. Method for the postdischarge surveillance of a patient for detecting a case of pneumonia, secondary bleeding, a wound healing problem, a pulmonary complication, a urinary tract infection or a thrombosis of the patient (1), for the implementation of which a data bank (13) is provided, arranged at a location other than the location (2) at which the patient (1) is based during the surveillance, having the following method steps:
- recording relevant data on the patient (1) for the postdischarge surveillance of the latter and
- interactively transmitting the relevant data to the data bank (13) by means of speech input by the patient.

2. Method for the postdischarge surveillance of a patient according to Claim 1, in which the data bank (13) is operated by a service provider (10).

3. Method for the postdischarge surveillance of a patient according to Claim 2, in which the service provider (10) is assigned a call center (9).

4. Method for the postdischarge surveillance of a patient according to one of Claims 1 to 3, in which the relevant data comprise a description of the general condition of the patient (1).

5. Method for the postdischarge surveillance of a patient according to one of Claims 1 to 4, in which the data bank (13) can be interrogated via the Internet.

6. Method for the postdischarge surveillance of a patient according to one of Claims 1 to 5, in which the data bank (13) automatically informs the patient (1) and/or at least one other person (4) if the relevant data are not received.

7. Method for the postdischarge surveillance of a patient according to one of Claims 1 to 6, in which the relevant data are evaluated, in particular automatically, by an evaluation device (14) assigned to the data bank (13).

8. Method for the postdischarge surveillance of a patient according to Claim 7, in which the data bank (13) automatically alerts the patient (1) and/or at least one other person (4) if the evaluated relevant data are critical.

9. Device for the postdischarge surveillance of a patient for detecting a case of pneumonia, secondary bleeding, a wound healing problem, a pulmonary complication, a urinary tract infection or a thrombosis of the patient (1), having
- a data bank (13) which is arranged at a location other than the location (2) at which the patient (1) is based during the surveillance and stores data relevant for the postdischarge surveillance of the patient (1) recorded during the postdischarge surveillance,
- means for speech input, which are designed to transmit data relevant to the data bank (13) interactively by speech,
- an evaluation device (14) assigned to the data bank (13) for the evaluation of the relevant data and
- an alarm device for generating an alarm signal if the evaluated relevant data are critical.

10. Device for the postdischarge surveillance of a patient according to Claim 9, in which the data bank (13) is configured in such a way that it automatically informs the patient (1) and/or at least one other person (4) if the relevant data are not received.

11. Method for the postdischarge surveillance of a patient (1) having the following method steps:
- recording, at the location (2) of the postdischarge surveillance, relevant data which are suitable for detecting a case of pneumonia, secondary bleeding, a wound healing problem, a pulmonary complication, a urinary tract infection or a thrombosis of the patient (1) during the postdischarge surveillance of the latter,
- interactively transmitting the relevant data via an information transmission network to a central data bank (13) by means of speech input by the patient,
- evaluating the relevant data using an evaluation device (14) assigned to the data bank (13).

12. Method according to Claim 11, in which the patient (1) and/or at least one other person (4) is automatically alerted if the evaluated relevant data are critical.

## Revendications

1. Procédé pour surveiller un patient après une hospitalisation afin de détecter une pneumonie, une hémorragie secondaire, un défaut de cicatrisation, une complication pulmonaire, une infection des voies urinaires ou une thrombose du patient (1), dont l'exécution prévoit une base de données (13) située à un endroit différent de l'endroit (2) où le patient (1) se trouve pendant la surveillance, le procédé comprenant les étapes suivantes :
- relever des données pertinentes du patient (1) pour sa surveillance après une hospitalisation, et
- transmettre de façon interactive les données pertinentes à la base de données (13) grâce à une saisie vocale par le patient.

2. Procédé pour surveiller un patient après une hospitalisation selon la revendication 1, d'après lequel la base de données (13) est gérée par un prestataire de services (10).

3. Procédé pour surveiller un patient après une hospitalisation selon la revendication 2, d'après lequel un centre d'appels (9) est associé au prestataire de services (10).

4. Procédé pour surveiller un patient après une hospitalisation selon l'une des revendications 1 à 3, d'après lequel les données pertinentes comprennent une description de l'état général du patient (1).

5. Procédé pour surveiller un patient après une hospitalisation selon l'une des revendications 1 à 4, d'après lequel la base de données (13) peut être interrogée par Internet.

6. Procédé pour surveiller un patient après une hospitalisation selon l'une des revendications 1 à 5, d'après lequel la base de données (13) informe automatiquement le patient (1) et/ou au moins une autre personne (4) quand les données pertinentes font défaut.

7. Procédé pour surveiller un patient après une hospitalisation selon l'une des revendications 1 à 6, d'après lequel les données pertinentes sont en particulier automatiquement exploitées par un dispositif d'exploitation (14) associé à la base de données (13).

8. Procédé pour surveiller un patient après une hospitalisation selon la revendication 7, d'après lequel la base de données (13) alerte automatiquement le patient (1) et/ou au moins une autre personne (4) quand les données pertinentes exploitées sont critiques.

9. Dispositif pour surveiller un patient après une hospitalisation pour détecter une pneumonie, une hémorragie secondaire, un défaut de cicatrisation, une complication pulmonaire, une infection des voies urinaires ou une thrombose du patient (1), le dispositif comportant
- une base de données (13) qui est à un endroit différent de l'endroit (2) où le patient (1) se trouve pendant la surveillance et qui, pendant la surveillance après une hospitalisation, enregistre des données pertinentes relevées pour surveiller le patient (1) après une hospitalisation,
- des moyens de saisie vocale conçus pour transmettre interactivement par la voix des données pertinentes à la base de données (13),
- un dispositif d'exploitation (14) associé à la base de données (13) et destiné à exploiter les données pertinentes et
- une installation d'alarme pour produire un signal d'alarme quand les données pertinentes et exploitées sont critiques.

10. Dispositif pour surveiller un patient après une hospitalisation selon la revendication 9, dans lequel la base de données (13) est conçue de telle manière qu'elle informe automatiquement le patient (1) et/ou au moins une autre personne (4) quand les données pertinentes font défaut.

11. Procédé pour surveiller un patient (1) après une hospitalisation, le procédé comportant les étapes suivantes :
- relever, à l'endroit (2) de la surveillance après une hospitalisation, des données pertinentes qui sont adaptées à détecter une pneumonie, une hémorragie secondaire, un défaut de cicatrisation, une complication pulmonaire, une infection des voies urinaires ou une thrombose du patient (1) pendant sa surveillance après une hospitalisation,
- transmettre de façon interactive les données pertinentes à une base de données (13) centrale grâce à une saisie vocale par le patient par l'intermédiaire d'un réseau de transmission des informations,
- exploiter les données pertinentes à l'aide d'un dispositif d'exploitation (14) associé à la base de données (13).

12. Procédé selon la revendication 11, d'après lequel le patient (1) et/ou au moins une autre personne (4) sont automatiquement alertés quand les données pertinentes exploitées sont critiques.
